# EUROPEAN PATENT SPECIFICATION

(11) **EP 1 377 298 B1**
(45) Date of publication and mention of the grant of the patent: **30.08.2006**
(21) Application number: 02727314.3
(22) Date of filing: 18.01.2002
(51) Int. Cl.: A61K 31/5685, A61P 5/24, A61K 45/06

(54) **EXEMESTANE FOR TREATING HORMONO-DEPENDENT DISORDERS**
EXEMESTAN ZUR BEHANDLUNG VON HORMONABHÄNGIGEN STÖRUNGEN
EXEMESTANE POUR TRAITER LES TROUBLES HORMONODEPENDANTS

(30) Priority: 26.01.2001 US 770911
(43) Date of publication of application: 07.01.2004
(73) Proprietor: Pfizer Italia S.r.l., 04010 Latina (IT); Pharmacia & Upjohn Company LLC, Kalamazoo, MI 49001 (US)
(72) Inventor: DI SALLE, Enrico, I-20124 Milano (IT); PISCITELLI, Gabriella, I-20135 Milano (IT); MASSIMINI, Giorgio, I-20081 Abbiategrasso (IT); PURANDARE, Dinesh, Bryn Mawr, PA 19010 (US); DEKONING, Gans, Hendrik, Mendham, NJ 07945 (US)
(74) Representative: Modiano, Micaela Nadia
(86) International application number: PCT/EP2002/000638
(87) International publication number: WO 2002/072106

(56) References cited:
- EP-A- 0 253 591
- WO-A-00/38730
- WO-A-01/87334
- WO-A-02/20020
- WO-A-95/01366
- US-A- 4 808 616
- US-A- 5 502 044
- "[Aromatase inactivator slows down endometriosis ]. AROMATASE-INAKTIVATOR BREMST ENDOMETRIOSE." ARZTLICHE PRAXIS GYNAKOLOGIE, (2002) -/3 (28)., XP008016170
- MILLER W.: "Proceedings of the International Symposium on Aromatase and its Inhibitors: New Biology and Clinical Perspectives. 3-6 September 1998, Prague, Czech Republic: Editorial." ENDOCRINE-RELATED CANCER, (1999) 6/2 (127-130)., XP008016173
- COMBS D.W.: "Recent developments in aromatase inhibitors." EXPERT OPINION ON THERAPEUTIC PATENTS, (1995) 5/6 (529-533)., XP008016169
- BOWDEN C.R. ET AL: "Aromatase inhibitors: 1992 patent activity." CURRENT OPINION IN THERAPEUTIC PATENTS, (1993) 3/6 (743-747)., XP008016171
- BULUN S E ET AL: "Estrogen production in endometriosis and use of aromatase inhibitors to treat endometriosis." ENDOCRINE-RELATED CANCER. ENGLAND JUN 1999, vol. 6, no. 2, June 1999 (1999-06), pages 293-301, XP008016200 ISSN: 1351-0088
- ZACCHEO T ET AL: "A new irreversible aromatase inhibitor, 6-methylenandrosta-1,4-diene-3,17 dione ( FCE 24304 ): antitumor activity and endocrine effects in rats with DMBA-induced mammary tumors." CANCER CHEMOTHERAPY AND PHARMACOLOGY, (1989) 23 (1) 47-50., XP008016172

## Description

### FIELD OF THE INVENTION

The present invention relates to medicaments for preventing and treating hormono-dependent disorders, in particular, estrogen-dependent disorders, selected from uterine fibroids and dysfunctional uterine bleeding.

### BACKGROUND OF THE INVENTION

Benign breast disease, or often called fibrocystic breast disease, appears to be dependent on ovarian steroids. See Jacquemier et al., Cancer, 49, 2534 (1982). Aromatase inhibitors have not been tried in this disease, but antiestrogens seem to be of benefit. See Ricciardi & Ianniruberto, Obstet. Gynecol., 54, 80 (1979).

Uterine fibroids, which appear in the reproductive years and regress after menopause, are the result of cellular proliferation and differentiation in the uterine tissue regulated by the ovarian steroids. At present, treatment of a patient suffering from uterine fibroids include surgery and administration of GnRH agonists.

Polycystic ovarian disease is one of the most common causes of infertility in women. The disease appears to result from an, abnormality in steroid metabolism, and the major form of therapy in this disease is the antiestrogan, olomiphene. See Yen, Clin. Endocrinol., 12, 177 (1980).

Fibrocystic mastopathy is a condition considered in the past to confer an increased risk for breast cancer. Reevaluation of the outcome of this disorder has concluded that the overall increased risk of 1.86 of developing breast cancer, estimated by pooling together many published series, was more likely due to the selection of patients than to the real malignant potential of the disease. The presence of proliferation with cell atypia on pathologic assessment, however, is associated with an increased risk for breast cancer, especially if the patient has a positive family history. Fibrocystic disease occurs more often among individuals 30 to 55 years of age, and is frequently identified by women as multiple, round lumps in one ar both breasts. The mammographic patterns of multiple areas of fobrosis and cysis are typical, but represent a difficult background for evaluation of an underlying neoplasia.

It is the object of the present invention to provide a medicament for preventing and treating estrogen dependent disorders selected from uterine fibroids and dysfunctional uterine bleeding,
said medicament being not as invasive as surgery and not characterized by the adverse side effects that accompany administration of drugs that suppress the activity of the ovaries.

"Aromatase-Inactivator bremst Endometriose", Arztliche Praxis Gynakologie, (2002)-/3(28), discloses exemestan to have a positive effect in the treatment of endometriosis, after seven months of treatment with 25mg/day.
WO 01/87334 discloses a method of treating a hormone-dependent disorder as breast cancer, cervical, ovarian and endometrial cancers and endometriosis, by administration of an aromatase inhibitor as exemestane and of an antibody against HER2.
WO 00/38730 discloses methods to treat or prevent neoplasia disorders using a combination of a cyclooxygenase-2 inhibitor and an antineoplastic agent. Miller W., "Proceedings of the International Symposium on Aromatase and its Inhibitors, New Biology and Clinical Perspectives, 3-6 September 1998, Prague, Czech Republic: Editorial "Endocrine-Related Cancer, (1999) 6/2 (127-130), discloses that excess estrogen production can result in several clinical disorders other than breast cancer, as endometriosis, precocious puberty, gynecomastia and hyperplastic breast lesions and that aromatase inhibitors represent a treatment option for endometriosis, the successful treatment of endometriosis with anastrozole being reported.
Combos D.W., "Recent developments in aromatase inhibitors", Expert Opinion on the Therapeutic Patents, (1995) 5/6 (529-533), discloses that aromatase inhibitors are reported to have potential in the treatment of diseases mediated by estrogen such as emdometriosis, uterine fibroids, endometrial cancer and benign prostatic hyperplasia, and indicates that exemestane is undergoing clinical trials for hormone dependent cancer.
Bowden C.R. et al., "Aromatase inhibitors: 1992 patent activity", Current Opinion in Therapeutic Patents, (1993) 3/6 (743-747) discloses that inhibition of aromatase provides a therapeutic approach to the treatment of estrogen dependent disease as endometriosis, uterine fibroids, breast cancer and endometrial cancer and indicates examestane as an example of steroidal inhibitors which act as suicide substrates for aromatase.

### SUMMARY OF THE INVENTION

A medicament for prevention and treatment of estrogen dependent disorders selected from uterine fibroids and dysfunctional uterine bleeding, is disclosed which comprises administering to a mammalian patient in need of such treatment an effective amount of the aromatase inactivator exemestane, alone or in combination with additional therapeutic agents.

### DETAILED DESCRIPTION OF THE INVENTION

The present invention relates to a medicament for preventing and treating estrogen dependent disorders selected from uterine fibroids and dysfunctional uterine bleeding,
which comprises administering to a mammal patient in need of such treatment an effective amount of exemestane, either alone or in combination with an additional therapeutic agents, thus achieving a therapeutic effect.

Therefore, the invention provides medicament for preventing and treating an estrogen dependent disorder selected from uterine fibroids and dysfunctional uterine bleeding,
in a mammal in need of such treatment, including humans, comprising administering to said mammal exemestane in amounts sufficient to achieve a therapeutically useful effect.

The invention also provides a medicament for preventing and treating an estrogen dependent disorder selected from uterine fibroids and dysfunctional uterine bleeding,
in a mammal in need of such treatment, including humans, comprising administering simultaneously, separately or sequentially to said mammal exemestane and another therapeutic agent, in amounts and close in time sufficient to achieve a therapeutically useful effect.

A further object of the present invention is to provide the use of exemestane in the manufacture of a medicament for preventing and controlling an estrogen dependent disorder selected from uterine fibroids and dysfunctional uterine bleeding.

The present invention also provides the use of exemestane in the manufacture of a medicament for preventing and controlling an estrogen dependent disorder selected from uterine fibroids, and dysfunctional uterine bleeding, in a patient undergoing a simultaneous, separate or sequential treatment with another therapeutic agent

The invention also provides a product containing exemestane and another therapeutic agent as a combined preparation for simultaneous, separate or sequential use in preventing and controlling an estrogen dependent disorder selected from uterine fibroids and dysfunctional uterine bleeding.

The combination preparation according to the invention can also include combination packs or compositions in which the constituents are placed side by side and can be administered simultaneously, separately of sequentially to one and the same human, being. Accordingly, exemestane and the other therapeutic agent may be present within a single or distinct container.
Accordingly, the invention also provides kits or single packages containing the pharmaceutical compositions useful for the combination treatment of the estrogen dependent disorder discussed above. The kits or packages may also contain instructions to use the pharmaceutical compositions in accordance with the present invention.
The inventors of the present invention have also found that prevention and control of the above mentioned estrogen-dependent disorders by combined administration of a therapeutically effective amount of exemestane and a therapeutically effective amount of another therapeutic agent, can produce a therapeutic affect which is greater than that obtainable by single administration of a therapeutically effective amount of either sole exemestane or the sole "additional" therapeutic agent. Namely, such combined therapy provides a synergistic or superadditive therapeutic effect.

Most importantly, they have found that such newly obtained therapeutic effect is not paralleled by the toxic effects, otherwise caused by single administration of either therapeutically effective amounts of exemestane or, of the "additional" therapeutic agent.

Product exemestane is compound 6-methylenandrost-1,4-diene-3,17-dione, which is known for instance from US Pat. No. 4,808,616.

The "additional" therapeutic agent for combination therapy with exemestane of the above mentioned estrogen-dependent disorders is for instance an agent selected from danazol,
a non-steroidal and compound (NSAID), a retinoid compound, a matrix metallo-protease inhibitor, an anti-estrogen, GnRH agonist or antagonist, a selective progestin receptor modulator (SPRM) and an angiogenesis inhibitor, or a mixture thereof.

A therapeutic agent mixture, according to the invention, which can be administered in combination with exemestane can comprise: one or more, preferably 2 to 4, in particular 2, therapeutic agents, as defined above.

Danazol, an androgen derivative which suppresses the pituitary-ovarian axis by inhibiting the release of GnRH, is well known in the art.

A non-steroidal anti-inflammatory compound (NSAID), according to the invention, is e.g. a compound selected from acetyl salicylic acid, indometacin, sulindac, phenylbutazone, diclofenac, fentiazac, ketorolac) piroxicam, tenoxicam, mecoxicam, meloxicam, cinnoxicam, ibufenac, ibuprofen, naproxen, ketoprofen, nabumetone, niflurmic acid and nimesulide, or a pharmaceutically acceptable salt thereof. Preferred NSAIDs are diclofenac, piroxicam, tenoxicam, mecoxicam, meloxicam, ibufenac, ibuprofen, naproxen and ketoprofen, or a pharmaceutically acceptable salt thereof.

Examples of retinoid compounds according to the invention include, for example, Accutane; Adapalene; Allergan AGN-193174; Allergan AGN-193676; Allergan AGN-193836; Allergan AGN-193109; Aronex AR-623; BMS-181162; Galderma CD-437; Eisai ER-34617; Etrinate; Fenretinide; Ligand LGD-1550; lexacalcitol; Maxia Pharmaceuticals MX-781; mofarotene; Molecular Design MDI-101; Molecular Design MDI-301; Molecular Design MDI-403; Motretinide; Eisai 4-(2-[5-(4-methyl-7-ethylbenzofuran-2-yl)pyrrolyl])benzoic acid; Johnson & Johnson N-[4-[2-thyl-1-(1H-imidazol-1-yl)butyl]phenyl]-2-benzothiazolamine;Soriatane; Roche SR-11262; Tocoretinate; Advanced Polymer Systems trans-retinoic acid; UAB Research Foundation UAB-8; Tazorac; TopiCare; Taiho TAC-101; and Vesanoid.

Examples of matrix metallo-protease inhibitors according to the invention include known:
1-cyclopropyl-N-hydroxy-4-[[4-[4-(trifluoromethoxy)phenoxy]phenyl]sulfonyl]-4-piperidinecarboxamide monohydrochloride;
N-hydroxy-1-(phenyhnethyl)-4-[[4-[4-(trifluoromethoxy)phenoxy]-1-piperidinyl]sulfonyl]-4-piperidinecarboxamide monohydrochloride;
N-hydroxy-1-(pyridinylmethyl)-4-[[4-[4-(trifluoromethyl)phenoxy]phenyl]sulfonyl]-4-piperidinecarboxamide dihydrochloride;
N-hydroxy-2,3-dimethoxy-6-[[4-[4-(trifluoromethyl)phenoxy]-1-piperidinyl]sulfonyl]-benzamide;
N-hydroxy-1-(4-pyridinylmethyl)-4-[[4-[4-(trifluoromethyl)phenoxy]phenyl] sulfonyl]-4-piperidinecarboxamide dihydrochloride;
N-hydroxy-1-(3-pyridinylmethyl)-4-[[4-[4-(trifluoromethyl)phenoxy]phenyl] sulfonyl]-4-piperidinecarboxamide dihydrochloride;
N-hydroxy-1-(2-pyridinylmethyl)-4-[[4-[4-(trifluoromethyl)phenoxy]phenyl]sulfonyl]-4-piperidinecarboxamide monohydrochloride;
British Biotech BB-2516 (marimastat), N4-[2,2-dimethyl-1-[(methylamino)carbonyl]-propyl]-N1,2-dihydroxy-3-(2-methylpropyl)-, [2S-[N4(R*), 2R*, 3S*]]-);
BMS 275291 disclosed in WO 97/19075;
Bayer Ag Bay-12-9566 (tanomastat), 4-[(4'-chloro[1,1-diphenyl]-4-yl)oxyl-2-[(phenylthio)methyl]butanoic acid;
Agouron Pharmaceuticals AG-3340, N-hydroxy-2,2'-dimethyl-4-[[4-(4-pyridinyloxy)phenyl]sulfonyl]-3-thiomorpholinecarboxamide;
CollaGenex Pharmaceuticals CMT-3 (metastat), 6-demethyl-6-deoxy-4-dedimethylaminotetracycline, batimastat (BB-94); and
Chiroscience D-2163, 2-[1S-([(2R,S)-acetylmercapto-S-phthalimido]pentanoyl-L-leucyl)amino-3-methylbutyl]imidazole.

An anti-estrogen, e.g. a selective estrogen receptor modulator (SERM), is preferably a SERM devoid of uterotrophic activity. Examples of SERMs, according to the invention, are tamoxifen, toremifene, arzoxifene, idoxifene, EM 800, fulvestrant and droloxifene.

Examples of GnRH (LHRH) agonists according to the invention are, e.g., leuprorelin, deslorelin, triptorelin, buserelin, nafarelin, goserelin, avorelin, histerelin, compound PTL 03001 (5-oxo-L-propyl-L-histidyl-L-tryptophyl-L-seryl-L-tyrosyl-D-tryptophyl-L-leucyl-L-arginyl-N-ethyl-L-prolinamide) (Peptech), compound AN 207 (6-[N6-[5-[2-[1,2,3,4,6,11-hexahydro-2,5,12-trihydroxy-7-mehoxy-6,11-dioxo-4-[[2,3,6-trideoxy-3-(2,3-dihydro-1H-pyrrol-1-yl).alpha.-L-lyxo-hexopyranosyl]oxy]-2-naphthacenyl]-1,5-dioxopentyl]-D-lysine]-,(2S-cis)-) (ASTA Medica Inc.), compound AN 238 L-threoninamide, N-[5-[2-[(2S,4S)-1,2,3,4,6,11-hexahydro-2,5,12-trihydroxy-7-methoxy-6,11-dioxo-4-[[2,3,6-trideoxy-3-(2,3-dihydro-1H-pyrrl-1-yl).alpha.-L-lyxo-hexopyranosyl]oxy]-2-naphthacenyl]-2-oxoethoxy]-1,5-dioxopentyl]-D-phenylalanyl-L-cysteinyl-L-tyrosyl-D-tryptophyl-L-lysyl-L-valyl-L-cysteinylcyclic (2.fwdarw.7)-disulfide (ASTA Medica Inc.) and compound SPD 424 (LHRH-hydrogel implant) (Shire Pharmaceuticals Group), or a pharmaceutically acceptable salt thereof.
Preferred examples are triptorelin, leuprorelin and goserelin, or a pharmaceutically acceptable salt thereof, in particular triptorelin or a pharmaceutically acceptable salt thereof, e.g. as triptorelin pamoate.

Examples of GnRH (LHRH) antagonists, according to the invention, are e.g. cetrorelix, abarelix, ramorelix, teverelix, ganirelix, compounds A 75998 (Acetyl-D-(2-naphthyl)alanyl-D-(4-chlorophenyl)alanyl-D-(3-pyridyl)alanyl-seryl-(N-methyl)tyrosyl-N6-(nicotinoyl)-D-lysyl-leucyl-N6-(isopropyl)lysyl-propyl-D-alaninamide) and A 84861 (Tetrahydrofuran-2-(S)-ylcarbonyl-glycyl-D-(2-naphthyl)alanyl-D-(4-cholro)phenylalanyl-D-(3-pyridyl)-alanyl-L-(N-methyl)tyrosyl-D-[N6-(3-pyridylcarbonyl)]lysyl-L-leucyl-L-(N6-isopropyl)lysyl-L-propyl-D-alanylamide)(Abbot Labs.), GnRH immunogen (Aphton Co.), compound T 98475 (Isopropyl 3-(N-benzyl-N-methylaminomethyl)-7-(2,6-difluorobenzyl)-4,7-dihydro-2-(4-isobutyrylaminophenyl)-4-oxothieno[2,3-*b*pyridine-5-carboxylate hydrochloride) (Takeda), and compound MI 1544 (Acetyl-D-tryptophyl-D-cyclopropyl-alanyl-D-tryptophyl-L-seryl-L-tyrosyl-D-lysyl-L-leucyl-L-arginyl-L-propyl-D-alaninamide), or a pharmaceutically acceptable salt thereof.
Preferred example is abarelix or a pharmaceutically acceptable salt thereof.

Examples of selective progestin receptor modulators (SPRMs), according to the invention, are e.g. dienogest or a pharmaceutically acceptable salt thereof.

An angiogenesis inhibitor is e.g. an αvβ3 integrin inhibitor, a protein kinase inhibitor, angiostatin, platelet factor 4 (endostatin), a VEGF inhibitor or thalidomide.

Vascular endothelial growth factor (VEGF) inhibitors and telomerase inhibitors are well known in the art. For instance, compounds SU 5416 and SU 6668, cited herein, are also VEGF inhibitors.

Moreover known VEGF inhibitors or antagonists are agents which suppress angiogenesis by reducing binding of VEGF to cellular receptors, including but not limited to, for example blocking monoclonal antibodies against the growth factor (e.g. rhuMAbVEGF, Ryan et al., Toxicol Pathol 1999, 27:78-86), against the receptor (e.g. DC101 and derivatives, Witte et al., Cancer Metastasis Rev 1998, 17:155-61), soluble forms of VEGF receptors (e.g. soluble Flt, Aiello et al., Proc Natl Acad Sci U S A 1995, 92:10457-61), or compounds which directly antagonise interactions between VEGF and cell surface receptors (e.g. Fairbrother et al., Biochemistry 1998,37:17754-64).

A protein kinase inhibitor, according to the invention, is for instance a tyrosine kinase inhibitor, in particular compound SU6668, i.e. 3-[4-(2-carboxyethyl-3,5-dimethylpyrrol-2-yl)methylidenyl]-2-indolinone, and compound SU5416, i.e. 3-[(2,4-dimethylpyrrol-5-yl)methylidenyl]-2-indolinone, which are known from WO 96/40116 and WO 99/61422.

Examples of αvβ3 integrin inhibitors are known:
Vitaxin antibody (Ixsys); Merck KgaA EMD-121974, cyclo[RGDF-N(Me)V-];
(10S)-10,11-dihydro-3-[3-(2-pyridinylamino)propoxy]-5H-dibenzo[a,d]cycloheptene-10-acetic acid;
(2S)-7[[(1H-benzimidazol-2-ylmethyl)methylamino]carbonyl]-2,3,4,5-tetrahydro-4-methyl-3-oxo-1H-1,4-benzodiazepine-2-acetic acid;
(2S)-2,3,4,5-tetrahydro-4-methyl-7-[[[(5-methyl-1H-imidazo[4,5-b]pyridin-2-yl]methyl]amino]carbonyl]-3-oxo-1H-1,4-benzodiazepine-2-acetic acid;
(bR)-b-[[[(3R)-2-oxo-3-[2-(5,6,7,8-tetrahydro-[1,8]-naphthyridin-2-yl)ethyl]1-1-pyrrolidinyl]acetyl]amino]-d-(1H-indol-3-yl)pentanoid acid; and
(3R)-N-[3-hydroxy-5-[(1,4,5,6-tetrahydro-5-hydroxy-2-pyrimidinyl)amino]benzoyl]-glycyl-3-(3-bromo-5-chloro-2-hydroxyphenyl)-b-alanine (compound SD 7784).

Angiostatin, endostatin and thalidomide are well known in the art. Pharmaceutically acceptable salts of the compound mentioned herein are well known in the art.

### Method and Administration

In effecting treatment of a patient in a therapy/prophylactic method according to the invention, exemestane and the other therapeutic agent can be administered in any form or mode which makes the compounds bioavailable in effective amounts, including oral and parenteral routes.

By the teams "controlling" and "treating" an estrogen dependent disorder, as used herein, is meant a method of achieving a therapeutically useful effect, in particular of curing such disorder.
The term "therapeutically useful effect", besides curing such disorders, also means giving relief from pain accompanying such disorders.

The term "close in time" means that in the combined method of treatment according to the invention, exemestane may be administered simultaneously with a further therapeutic agent or the compounds may be administered sequentially, in either order, to achieve a therapeutic effect.
By the term "administered" or "administering" as used herein is meant any acceptable manner of administering a drug to a patient which is medically acceptable including parenteral and oral administration.

By "parenteral" is meant intravenous, subcutaneous, intra-nasal, pulmonary, intradermal or intramuscular administration.

Oral administration includes administering exemestane of the constituents of the combined preparation in a suitable oral form such as, e.g., tablets, capsules, suspensions, solutions, emulsions, powders, syrups and the like.

The actual preferred use and order of administration of the combined preparations of the invention may vary according to, inter alia, the particular pharmaceutical formulation of exemestane being utilized, the particular pharmaceutical formulation of the other therapeutic being utilized, the particular estrogen-dependent disorder to be prevented or treated and the particular patient being treated.

In the combined medicament for prevention or treatment according to the subject invention, exemestane may be administered simultaneously with the other therapeutic agent or the compounds may be administered sequentially, in either order. Preferably the compounds are administered sequentially. In particular when the combination treatment comprises exemestane and a GnRH agonist or antagonist, preferably, the compounds are administered in such a way that in the patient both inhibition of hormone output of her ovaries and inhibition/inactivation of aromatase enzyme are contemporaneously provided, and thus a therapeutic useful effect is achieved.

### Dosage

The dosage ranges for the administration of the combined preparation may vary with the age, condition and extent of the disease in the patient and can be determined by one of skill in the art.

The dosage regimen must therefore be tailored to the particular of the patient's conditions, response and associate treatments in a manner which is conventional for any therapy, and may need to be adjusted in response to changes in conditions and/or in light of other clinical conditions.

According to the medicament for preventing and treating estrogen dependent disorders in mammals, provided the present invention, exemestane for instance can be administered orally in a dosage range varying from 2.5 mg daily to 600 mg daily, in particular from 10 to 50, more preferably from 10 to 25 mg daily, or parenterally in a dosage ranging from 50 to 500 mg per injection.

As a preferred embodiment of the invention, exemestane may be orally administered in the form of a complex with cyclodextrins, in particular exemestane/β-cyclodextrin complex, at a daily dosage ranging from 10 to 20 mg, preferably 15 or 20 mg.

The effective therapeutic amounts of the other therapeutic agents to be used in combination with exemestane, according to the invention, are in general those commonly used in therapy for such compounds. More specifically, a therapeutically effective amount of another therapeutic agent means an amount of a compound, which when administered in combination with exemestane, is effective to prevent or treat estrogen- dependent disorders, as herein defined.

Determination of a therapeutically effective amount is well within the capability of those skilled in the art.

For instance an effective amount of compound SU 5416 or SU 6668 is an amount in accordance with the teaching of WO 99/61422.

An effective amount of compound SD 7784 is from 10 to 300 mg/kg, preferably per os, in particular from 20 to 200 mg/kg.
An effective amount of thalidomide may be in the range of 100 to 400 mg/day.

An anti-estrogen can be administered in a dosage according to the common practice, e.g. in a dosage of 0.1 to 30 mg/Kg body weight per day.

An effective amount of tamoxifen may be in the range of 10 to 40 mg/day. An effective amount of fulvestrant may be in the range of 50 mg to 300mg/day i.m., in particular of 100 to 250 mg/day i.m.

An effective amount of raloxifen may be in the range of 5 to 350 mg/day, in particular 60 mg/day.

An effective amount of a COX-2 inhibitor may be in the range of 0.1 to 2000 mg, preferably in the range of 0.5 to 500 and most preferably between 1 and 200 mg. In particular as to celecoxib, rofecoxib, parecoxib and valdecoxib, a daily dosage of 0.01 to 100 mg/Kg body weight, preferably between 0.1 and 50 mg/Kg body weight may be appropriate. The daily dosage can be administered in one to four doses per day.

More particularly, as to celecoxib a dosage from 50 to 500 mg, in particular 200 mg, once or twice a day may be appropriate.

As to rofecoxib the dosage normally ranges from 12.5 to 50 mg/day. The route of administration is preferably systemic e.g. oral or parenteral, in particular intravenous or intramuscularly.

Therapeutic dosages for SPRMs range between 2 to 50 mg/day.

Therapeutic dosages for GnRH agonists/antagonists like leuprolide are administered i.m. in doses varying from 1.5 to 15 mg, preferrably around 3.75 mg per month or 12.75mg per 3 months.

Goserelin can be administered as goserelin acetate by subcutaneous administration of slow release goserelin at a dosage from 3 to about 12 mg.

Triptorelin can be administered for instance as triptorelin pamaote by intramuscular administration of a sustained release formulation, in such a way that there is an interval from about 1 to 4 months between each administration and at a dosage from 3 to 20 mg. In particular triptorelin pamoate can be administered intramuscularly in the form of microparticles as described in US Pat. No. 5,225,205 and US Pat. No. 5,776,885, and more specifically as 1-month depot formulation 3.75 mg.

An effective amount of a NSAID, according to the invention is generally the one commonly used in therapy for such compound. For instance an effective mount of naproxen may be in the range of 300 mg to 750 mg once or twice a day.
An effective amount of proxicam may be in the range of 15 mg to 50 mg once or twice a day.

An effective amount of acetyl salioylio acid may be in the range of 150 to 1000 mg once or twice a day.

According to a preferred feature of the invention it is here provided a medicament for treating and preventing an estrogen dependent disorder selected from uterine fibroids and dysfunctional uterine bleeding.
in a mammal in need of such treatment, including humans, comprising administering simultaneously, separately or sequentially to said mammal exemestane and a GnRH agonist or antagonist selected from triptorelin, cetrorelix, and leuprolide, in particular triptorelin and leuprolide, in amounts and close in time sufficient to produce a therapeutically useful effect.
According to such preferred features exemestane is administered orally at (25 mg/day; triptorelin and leuprolide one or every three months at a dose of 3 or 20 mg, respectively, in particular of 3.75 or 12.75 mg, respectively.

According to a further preferred feature of the provided the use of exemestane in the manufacture of a medicament for preventing or controlling an estrogen dependent disorder selected from uterine fibroids, and dysfunctional uterine bleeding,
in a patient undergoing a simultaneous, separate or sequential treatment with a GnRH agonist or antagonist selected from triptorelin, cetrorelix, and leuprolide, in particular triptorelin and leuprolide more preferably triptorelin.

As an example a kit according to the present invention provides an exemestane 25 mg oral or 50-500 mg parenteral composition and a triptorelin depot formulation 3.75 mg.

A pharmaceutically composition containing exemestane and/or another therapeutic agent according to the invention can be prepared according to well known techniques to those skilled in the art.

A pharmaceutical composition for intramuscular administration containing triptorelin pamoate in the form of a depot formulation can be prepared for instance as described in US Pat. No. 5,225,205 and US Pat. No. 5,776,885.

A pharmaceutical composition containing exemestane can be prepared according to US Pat. No. 4,808,616. In particular an exemestane/β-cyclodextrin complex formulation can be obtained as follows:
Exemestane 20 mg Tablet

| | | |
|---|---|---|
| Composition: | exemestane | 20.00 mg |
| | Beta-cyclodextrin | 178.00 mg |
| | Avicel PH101 | 75.00 mg |
| | Explotab | 24.00 mg |
| | Magnesium stearate | 3.00 mg |

According to methods well known in the art an exemestane/cyclodextrin kneaded system can be prepared.

## Claims

1. Use of exemestane in the manufacture of a medicament for preventing and controlling an estrogen dependent disorder selected from uterine fibroids and dysfunctional uterine bleeding.

2. The use according to claim 1, wherein when administered orally the amount of exemestane in the medicament is from 2.5 mg to 600 mg daily.

3. The use according to claim 1, wherein when administered orally the amount of exemestane in the medicament is from 10 mg to 50 mg daily.

4. The use according to claim 1, wherein when administered orally the amount of exemestane, in the medicament is from 10 mg to 25 mg daily.

5. The use according to claim 1, wherein when administered parenterally the amount of exemestane in the medicament is from 50 mg to 500 mg.

## Patentansprüche

1. Verwendung von Exemestan in der Herstellung eines Medikaments zur Verhinderung und Eindämmung einer Östrogenabhängigen Krankheit, gewählt aus Gebärmutter-Fibromen und dysfunktioneller Gebärmutter-Blutung.

2. Die Verwendung gemäß Anspruch 1, worin die Menge von Exemestan in dem Medikament, wenn es oral verabreicht wird, von 2,5 mg bis 600 mg täglich ist.

3. Die Verwendung gemäß Anspruch 1, worin die Menge von Exemestan in dem Medikament, wenn es oral verabreicht wird, von 10 mg bis 50 mg täglich ist.

4. Die Verwendung gemäß Anspruch 1, worin die Menge von Exemestan in dem Medikament, wenn es oral verabreicht wird, von 10 mg bis 25 mg täglich ist.

5. Die Verwendung von Anspruch 1, worin die Menge von Exemestan in dem Medikament, wenn es parenteral verabreicht wird, von 50 mg bis 500 mg ist.

## Revendications

1. Utilisation d'exémestane dans la production d'un médicament destiné à la prévention et la lutte contre un trouble dépendant d' oestrogènes, choisi entre des fibromes utérins et un saignement utérin dysfonctionnel.

2. Utilisation suivant la revendication 1, dans laquelle, lors de l'administration par voie orale, la quantité d'exémestane dans le médicament est de 2,5 mg à 600 mg par jour.

3. Utilisation suivant la revendication 1, dans laquelle, lors de l'administration par voie orale, la quantité d'exémestane dans le médicament est de 10 mg à 50 mg par jour.

4. Utilisation suivant la revendication 1, dans laquelle, lors de l'administration par voie orale, la quantité d'exémestane dans le médicament est de 10 mg à 25 mg par jour.

5. Utilisation suivant la revendication 1, dans laquelle, lors de l'administration par voie parentérale, la quantité d'exémestane dans le médicament est de 50 mg à 500 mg.
